# EUROPÄISCHE PATENTANMELDUNG

(11) **EP 0 725 065 A2**
(43) Veröffentlichungstag der Anmeldung: **07.08.1996**
(21) Anmeldenummer: 96100819.0
(22) Anmeldetag: 20.01.1996
(51) Int. Cl.: C07D 251/54, C08K 5/3492, C07C 279/02

(54) **Verfahren zur Herstellung von Guanidin- oder Melaminphosphaten**

(30) Priorität: 06.02.1995 AT 203/95
(71) Anmelder: DSM Chemie Linz GmbH, 4021 Linz (AT)
(72) Erfinder: Horacek, Heinrich, Dr., A-4048 Linz/Puchenau (AT); Prinz, Christian, A-4060 Leonding (AT)
(74) Vertreter: Kunz, Ekkehard, Dr.

(57) **Zusammenfassung**

Verfahren zur Herstellung von Guanidin- oder Melaminphosphaten durch Erhitzen eines Gemisches aus Guanidincarbonat bzw. Melamin mit einem Ammoniumphosphat auf 150 bis 500°C für 10 Minuten bis 5 Stunden und anschließendem Abkühlen.

## Beschreibung

Guanidin- und Melaminphosphate gewinnen immer mehr als halogenfreie Flammhemmer an Bedeutung. So ist beispielsweise in US 4,010,137 die Verwendung von kondensierten Melaminphosphaten, wie etwa Dimelaminpyrophosphat, als Flammhemmer für Polyolefine beschrieben. Die Verwendung von Guanidinphosphaten, wie etwa Diguanidinpyrophosphat, beispielsweise zur Flammfestausrüstung von Zellulosetextilien ist unter anderem aus US 2.549.059 bekannt.

Bisher erfolgte die Herstellung dieser Verbindungen mittels Naßverfahren, beispielsweise durch Umsetzung von Phosphorsäuren oder ihren Salzen mit Guanidinderivaten bzw. mit Melamin (US 2.549.059, EP 0 413 376) in wäßrigem Medium. Der Nachteil dieser Naßverfahren liegt zu einem Teil in der aufwendigen Aufarbeitung, wie etwa Fest-Flüssigtrennung, Trocknung sowie Mahlung, und zum anderen Teil in der starken Verunreinigung der so hergestellten Verbindungen mit Nebenprodukten. So enthält beispielsweise ein analog EP 0 413 376 hergestelltes Dimelaminpyrophosphat bis zu 50 Gew.% an Melaminorthophosphat.

Aufgabe der vorliegenden Erfindung war es demnach ein neues Verfahren zu finden, das die Herstellung von Guanidin- oder Melaminphosphaten auf einfache Weise und unter Vermeidung von Nebenproduktbildung gewährleistet. Unerwarteterweise konnte diese Aufgabe durch ein Röstverfahren ausgehend von Ammoniumphosphaten gelöst werden.

Gegenstand der vorliegenden Erfindung ist demnach ein Verfahren zur Herstellung von Guanidin- oder Melaminphosphaten, das dadurch gekennzeichnet ist, daß ein Gemisch aus Melamin bzw. Guanidincarbonat mit einem Ammoniumphosphat auf eine Temperatur von 150 bis 500°C erhitzt und für 10 Minuten bis 5 Stunden in diesem Temperaturbereich gehalten wird, worauf auf Raumtemperatur abgekühlt und das gewünschte Endprodukt in Pulverform erhalten wird.

Nach dem erfindungsgemäßen Verfahren können sowohl Guanidin- als auch Melaminphosphate hergestellt werden. Unter Guanidin- bzw. Melaminphosphaten sind dabei Verbindungen zu verstehen, die durch Umsetzung von Melamin bzw. Guanidincarbonat mit einem Ammoniumphosphat erhalten werden, wobei das Verhältnis von Melamin bzw. Guanidin zu Phosphorsäure im jeweiligen Endprodukt variiert.

Ausgehend von einem Melamin- bzw. Guanidin-Phosphorsäure-Verhältnis im Endprodukt von 1 : 1, können somit durch Verwendung eines Melamin- bzw. Guanidin- oder eines Phorsphorsäureüberschusses unterschiedliche Verbindungen erhalten werden. Beispiele dafür sind primäres oder sekundäres Guanidinphosphat, Diguanidinpyrophosphat, Melaminphosphat, Dimelaminpyrophosphat, Dimelaminphosphat oder Melaminpolyphosphat. Bevorzugt werden nach dem erfindungsgemäßen Verfahren sekundäres Guanidinphosphat, Melaminphosphat und Melaminpyrophosphate hergestellt. Geeignete Ausgangsprodukte für das erfindungsgemäße Verfahren sind Melamin, Guanidincarbonat, sowie Ammoniumphosphate.

Als Ammoniumphosphate kommen dabei Diammoniumhydrogenphosphat, Triammoniumphosphat und Ammoniumdihydrogenphosphat in Frage. Bevorzugt wird Ammoniumdihydrogenphosphat verwendet. Die Ausgangsverbindungen werden in den zur Erzielung des gewünschten Melamin- bzw. GuanidinPhosphorsäure-Verhältnisses erforderlichen äquimolaren Mengen vermengt, wobei eine möglichst homogene Mischung erhalten werden sollte. Bevorzugt werden die Ausgangsprodukte deshalb vor dem Mischen fein vermahlen. Die Mischung der pulverförmigen Ausgangsprodukte kann jedoch zuerst auch in ein Granulat überführt werden. Die Mischung der jeweiligen Ausgangsverbindungen bzw. das Granulat wird sodann auf eine Temperatur zwischen 150 und 500°C erhitzt. Bevorzugt wird auf eine Temperatur zwischen 200 und 350°C erhitzt. Die Temperatur wird für die Dauer von 10 Minuten bis zu 5 Stunden, bevorzugt 30 Minuten bis zu 3 Stunden in diesem Temperaturbereich gehalten. Die Temperatur kann dabei über den ganzen Zeitraum konstant gehalten werden, sie kann jedoch auch innerhalb des oben definierten Temperaturbereiches nach einem Temperatur-Zeit-Programm gezielt verändert werden.
Anschließend wird auf Raumtemperatur abgekühlt bzw. abkühlen gelassen, worauf die gewünschte Melamin- bzw. Guanidinphosphatverbindung in kristalliner Form bzw. als Pulver, beispielsweise in freifließender oder in agglomerierter Form, erhalten wird.

Die bei dieser Reaktion anfallenden Abspaltprodukte sind Ammoniak, Wasser und, bei Einsatz von Guanidincarbonat, CO₂. Das abgegebene Ammoniak bzw. Ammoniak-CO₂-Gemisch kann in Phosphorsäure aufgefangen werden, wobei sich Ammoniumdihydrogenphosphat, gegebenenfalls unter Entweichen von CO₂, bildet, das wieder als Ausgangsprodukt eingesetzt werden kann.

Zur Durchführung des erfindungsgemäßen Verfahrens eignen sich heiz- und rührbare Reaktoren wie etwa beheizte Mischer, Draistrockner, Wirbelschichttrockner sowie Drehrohröfen. Bevorzugt wird das Verfahren in Drehrohröfen durchgeführt.
Durch das erfindungsgemäße Verfahren werden Melamin- oder Guanidinphosphatverbindungen in hoher Reinheit erhalten. Die so hergestellten Verbindungen eignen sich zur Verwendung als Flammhemmer. Ein besonderer Vorteil der erfindungsgemäß hergestellten Verbindungen ist, daß sie besonders arm an gasförmigen Spaltprodukten sind, wodurch sie sich besonders zur Einarbeitung in Kunststoffe mittels Extrusion eignen.

Ein weiterer Vorteil dieses Trockenverfahrens ist es, daß andere feste Zuschläge, wie Rieselhilfen, Wachse, Dispergiermittel, Agglomeriermittel, Farbpigmente, Harze und Koflammhemmer zugesetzt werden können. Ebenso erlaubt es die Herstellung von Mischsalzen.

Zuschläge der vorhin erwähnten Art sind Dispergiermittel wie Phosphorsäureester, Fettsäuremonoethanolamide, Aerosile, Wachse, Agglomeriermittel wie Harze, beispielsweise Orgasol 2001, 2002, 3501 der Fa. Elf Atochem, Rieselhilfen wie Zinkstearat oder Calziumstearat, Koflammhemmer wie Arylphosphate und Phosphonate.

### Beispiel 1:

In einem Drehrohrofen mit den Abmessungen 8 m Länge und 1,5 m Druchmesser, der im Inneren verstellbare Schaufeln besitzt, wurde ein homogenes Gemisch aus 230 Gewichtsteilen Ammoniumdihydrogenphosphat und 252 Gewichtsteilen Melamin mit einer Geschwindigkeit von 500 kg/h zudosiert. Die Schaufelstellung wurde so gewählt, daß die Verweilzeit 1 Stunde betrug.
Nach einer Verweilzeit von 1 Stunde betrug die Gewichtsabnahme 10,8 Gew.%. Anschließend wurde das pulverförmige Endprodukt analysiert.
Das Endprodukt war gemäß Elementaranalyse und DSC-Untersuchungen reines Dimelaminpyrophosphat. Das abgegebene Ammoniak wurde in Phosphorsäure aufgefangen, wobei sich wieder Ammoniumdihydrogenphosphat bildete.

### Beispiel 2:

Im gleichen Drehrohrofen wurde ein homogenes Gemisch aus 3 kg Aerosil 200 180 kg Guanidincarbonat mit 115 kg Ammoniumdihydrogenphosphat analog zu Beispiel 1 gefahren. Die Verweilzeit betrug 2 Stunden und der Gewichtsverlust 26,8 Gew.%. Gemäß Elementaranalyse und DSC-Untersuchung bestand das Endprodukt aus reinem sekundären Guanidinphosphat. Das abgegebene Ammoniak-CO₂-Gemisch wurde in Phosphorsäure aufgefangen, wobei sich wieder Ammoniumdihydrogenphosphat bildete und CO₂ entwich. Aufgrund der Rieselfhilfe Aerosil 200 war das erhaltene Pulver besonders fein und gut rieselfähig.

### Beispiel 3:

In einem heizbaren Turbulentmischer mit einem Nutzvolumen von 100 l wurden 12,6 kg Melamin, 15,5 kg Ammoniumdihydrogenphosphat und 2,8 kg Polyamidpulver während 1 Stunde bei einem Vakuum von 0,25 bar und bei einer Temperatur von 250°C gut gemischt.
Anschließend wurde auf Raumtemperatur abgekühlt und das agglomerierte Endprodukt analysiert.

Es bestand aus ca. 0,5 mm staubfreien Agglomeraten, die sich aus 90 % Dimelaminpyrophosphat und 10 % Polyamid zusammensetzen.

### Beispiel 4:

Im gleichen Turbulentmischer wurde ein Gemisch aus 15,5 kg Ammoniumdihydrogenphosphat 6,3 kg Melamin, 9 kg Guanidincarbonat und 0,3 kg Luwax EA S1 dosiert.
Die Mischzeit betrug bei 250°C 2 Stunden, dabei erfolgte eine Gewichtsabnahme um 18,6 %.
Man erhielt ein Mischsalz mit feiner Körnung und besonders guter Dispergierbarkeit in thermoplastischen Kunststoffen.

### Beispiel 5:

Im Mischer des Beispiels 3 wurde ein Gemisch aus 11,5 kg Ammoniumdihydrogenphsophat, 12,6 kg Melamin und 2,15 kg Triarylphosphat 1 Stunde bei 250°C erhitzt.
Nach dem Abkühlen wurde eine gut rieselnde, nicht staubende Mischung aus 90 % Dimelaminpyrophosphat und 10 % Triarylphosphat erhalten.

## Patentansprüche

1. Verfahren zur Herstellung von Guanidin- oder Melaminphosphaten, dadurch gekennzeichnet, daß ein Gemisch aus Melamin bzw. Guanidincarbonat mit einem Ammoniumphosphat auf eine Temperatur von 150 bis 500°C erhitzt und für 10 Minuten bis 5 Stunden in diesem Temperaturbereich gehalten wird, worauf auf Raumtemperatur abgekühlt und das gewünschte Endprodukt in Pulverform erhalten wird.

2. Verfahren nach Anspruch 1, dadurch gekennzeichnet, daß Melaminphosphat, Melaminpyrophosphate oder sekundäres Guanidinphosphat hergestellt werden.

3. Verfahren nach Anspruch 1, dadurch gekennzeichnet, daß als Ammoniumphosphat Diammoniumhydrogenphosphat, Triammoniumphosphat oder Ammoniumdihydrogenphosphat eingesetzt wird.

4. Verfahren nach Anspruch 1, dadurch gekennzeichnet, daß als Ammoniumphosphat Ammoniumdihydrogenphosphat eingesetzt wird.

5. Verfahren nach Anspruch 1, dadurch gekennzeichnet, daß der Temperaturbereich zwischen 200 und 350°C liegt.

6. Verfahren nach Anspruch 1, dadurch gekennzeichnet, daß das Gemisch aus Guanidincarbonat bzw. Melamin und Ammoniumphosphat für eine Dauer von 30 Minuten bis. zu 3 Stunden in dem Temperaturbereich gehalten wird.

7. Verfahren nach Anspruch 1, dadurch gekennzeichnet, daß die Reaktion in einerm heiz- und rührbaren Reaktor durchgeführt wird.

8. Verfahren nach Anspruch 7, dadurch gekennzeichnet, daß als heiz- und rührbarer Reaktor ein beheizter Mischer, ein Draistrockner, ein Wirbelschichttrockner oder ein Drehrohrofen verwendet wird.
